# EUROPEAN PATENT APPLICATION

(11) **EP 3 782 989 A1**
(43) Date of publication of application: **24.02.2021**
(21) Application number: 19193476.9
(22) Date of filing: 23.08.2019
(51) Int. Cl.: C07D 233/64, A61P 25/20, A61K 31/4174

(54) **DETOMIDINE HYDROCHLORIDE MONOHYDRATE**

(71) Applicant: Sachse, Andreas, 10709 Berlin (DE)
(72) Inventor: Sachse, Andreas, 10709 Berlin (DE)
(74) Representative: Dr. Klemens Schubert Patentanwalt

(57) **Abstract**

The present invention relates to detomidine hydrochloride monohydrate, a method for the production of the same and to the use of the same as a medicament, that is for use as anesthetic, analgesic, muscle relaxant, sedative and any other condition susceptible to treatment with an alpha₂-adrenoreceptor agonist in a mammal.

Detomidine hydrochloride monohydrate described herein represents the pure monohydrate form which additionally shows a high purity reflected the very low content of impurities and especially residual solvents. Correspondingly, detomidine hydrochloride monohydrate described herein, shows a very high storage stability which is useful for the production of pharmaceutical compositions for the treatment of pathological conditions.

## Description

### Technical Field

The present invention relates to detomidine hydrochloride monohydrate, a method for the production of the same and to the use of the same as a medicament, that is for use as anesthetic, analgesic, muscle relaxant, sedative and any other condition susceptible to treatment with an alpha₂-adrenoreceptor agonist in a mammal.

### Background Art

Detomidine hydrochloride (1H imidazole,4-[(2,3-dimethylphenyl)methyl]-hydrochloride (CAS Number: 90038-01-0) is a synthetic alpha₂-adrenoreceptor agonist with sedative and analgesic properties widely used for sedation of large animals like horses and cattle. This substance displays various other pharmacologic effects related to the cardiovascular and respiratory system as well as on muscles. Detomidine hydrochloride is available as a parenteral solution with 10 mg/ml as active ingredient which is indicated for use as a sedative and analgesic to facilitate minor surgical and diagnostic procedures in mature horses and yearlings (e.g. DORMOSEDAN®). Furthermore, detomidine hydrochloride is supplied as an oromucosal (i.e. sublingual) gel (e.g. DORMOSEDAN GEL®) with 7.6 mg/ml as active ingredient which is indicated for sedation and restraint in horses.
Further details regarding the clinical pharmacology and side effects as well as contraindications for this drug substance (i.e. active pharmaceutical ingredient) can be found in: Veterinary Psychopharmacology; Sharon L. et al., 2nd edition (2019), Wiley & Sons (pages 161 - 162). According to these authors detomidine has not been used in humans to date.
*Detomidini hydrochloridum ad usum veterinarium* is included in the EUROPEAN PHARMACOPOEIA (Ph. Eur. 9.0) but currently not included in the United States Pharmacopoeia (USP). It has to be noted that in the absence of a statement regarding a specific hydrate form, like a degree of hydration or mono-, di-, etc., in the title of the monograph - as is the case for detomidine hydrochloride - the anhydrous form is indicated for this substance.
According to a prior version of the respective monograph, namely Ph. Eur. 8.0, the substance exists as a white or almost white, hygroscopic, crystalline powder. The substance is soluble in water, freely soluble in ethanol (96 %), very slightly soluble in methylene chloride and practically insoluble in acetone. The molecular weight (Mᵣ) amounts to 222.7. The melting point (mp) is specified at about 160 °C. In the current monograph (Ph. Eur. 9.0) the content of detomidine hydrochloride is specified at 99.0 % to 101.0 percent (anhydrous substance).

In the current monograph (Ph. Eur. 9.0) the content of detomidine hydrochloride is specified at 99.0 % to 101.0 % (anhydrous substance).
The current monograph includes the three following known impurities:
Impurity A: (RS)-(2,3-dimethylphenyl) (1H-imidazol-4-yl)-methanol
Impurity B: (RS)-(1-benzyl-1H-imidazol-5-yl)(2,3-dimethylphenyl)-methanol
Impurity C: 4-[(2,3-dimethylcylohexyl)methyl]-1H-imidazole
The related substances are specified at ≤ 0.20 % for any unspecified impurities and ≤ 0.5 % for total impurities with a reporting threshold of 0.10 %.
The water content of detomidine hydrochloride as determined by Karl Fischer (KF) titration is limited to ≤ 2.0 % for release as well as shelf-life testing. As detomidine hydrochloride is hygroscopic, the compound has to be stored in airtight containers.

A synthesis method for detomidine was disclosed in US 4,584,383.
Specific details on the last two steps of a synthesis method for detomidine hydrochloride (including a reaction scheme) were published in Drugs Future 10, 17 (1985).

Detomidine hydrochloride is known to exist in two crystalline forms, namely the anhydrous form, as described above, and the monohydrate form B (Mᵣ: 240.7, CAS Number: 90038-00-9) which can easily interconvert, depending on ambient temperature and air humidity (Veldre, K. et al., Eur. Journ. Pharm. 44, 273-280 (2011)). At 80 % air humidity and room temperature the monohydrate is reversibly formed. The theoretical water content of detomidine hydrochloride monohydrate amounts to 7.48 %.

To date, all commercially available (i.e. veterinary) drug products (i.e. parenteral solutions and oromucosal gels) only contain the anhydrous form. In general, hygroscopic substances like detomidine hydrochloride tend to absorb moisture so that they have to be protected from a humid environment during production and storage of the drug substance and corresponding drug product to avoid an inacceptable uptake of water. It has to be noted that such uptake during storage will reduce the content of the drug substance so that this would have to be taken into consideration during production of the corresponding drug product, like pharmaceutical preparation.

The problem to be solved is to provide a pure and stable active pharmaceutical ingredient (API), namely detomidine hydrochloride monohydrate, that can advantageously be used for the production of pharmaceutical compositions comprising the active pharmaceutical ingredient detomidine hydrochloride.

### Summary of Invention

One object of the present invention is the provision of a pure and stable form of detomidine hydrochloride, namely detomidine hydrochloride monohydrate (DHM).
DHM according to the present invention represents the pure monohydrate and does not show any significant conversion to the anhydrate during storage.
DHM according to the present invention has a water content in the range of 7.4 to 7.6 % (w/w), a residual solvent content of less than 100 ppm for any of the detected single solvents and a HPLC purity equal to or higher than 99.8 %.

DHM according to the present invention does not show any significant changes in water content or other relevant specification parameters during storage. Preferred according to the present invention is DHM, wherein the storage stability at 25 °C and 60 % relative humidity is equal to or higher than 48 months. DHM according to the present invention does not require use of airtight containers for storage.

Another object of the present invention is a method for reproducible production of detomidine hydrochloride monohydrate which involves recrystallization of the initial detomidine hydrochloride raw product from the last step of the overall synthesis from water which results in a product of high purity in high yields. The method according to the invention can be performed in a standard production environment under ambient conditions without the need for harsh drying conditions, elaborate handling or humidity controls. Furthermore, this method does not require use of organic solvents during the last purification (i.e. recrystallization) step.

DHM according to the present invention may advantageously be used in pharmaceutical preparations for veterinary or human use via parenteral, oral or topical routes of application. Thus, one object of the present invention is DHM for use as a medicament.

A further object of the present invention is detomidine hydrochloride monohydrate for use as anaesthetic, analgesic, muscle relaxant, sedative and any other application susceptible to treatment with an alpha2-adrenoreceptor agonist in a mammal.

One object of the present invention is detomidine hydrochloride monohydrate, wherein the water content is in the range of 7.2 to 7.8 % (w/w). According to the invention it is preferred that detomidine hydrochloride monohydrate has a water content that is in the range of 7.4 to 7.6 % (w/w).

According to the invention it is especially preferred that the water content is in the range of 7.45 to 7.51 % (w/w).

Preferred according to the invention is also detomidine hydrochloride monohydrate, wherein the residual solvent content is less than 100 ppm for any of the detected single solvents.
Especially preferred is a residual solvent content that is equal to or less than 50 ppm for any of the detected single solvents.

According to the invention it is also preferred that detomidine hydrochloride monohydrate has a HPLC purity that is equal to or higher than 99.8 %.

Especially preferred is detomidine hydrochloride monohydrate, wherein the content of (RS)-(2,3-dimethylphenyl)(1H-imidazol-4-yl)-methanol, (RS)-(1-benzyl-1H-imidazol-5-yl)(2,3-dimethylphenyl)-methanol and 4-[(2,3-dimethylcylohexyl)methyl]-1H-imidazole as impurities is less than 0.1 % each. The herein mentioned impurities are impurities A, B and C as described above and as given in the state of the art.

Preferred according to the present invention is detomidine hydrochloride monohydrate, wherein the storage stability at 25 °C and 60 % relative humidity is equal to or higher than 36 months.
Especially preferred is a detomidine hydrochloride monohydrate, according to the invention, wherein the storage stability at 25 °C and 60 % relative humidity is equal to or higher than 48 months.

Another object of the present invention is detomidine hydrochloride monohydrate, obtainable by
a) providing detomidine hydrochloride raw product, wherein the raw product is synthesized from (RS)-(3-Benzyl-3*H*-imidazol-4-yl)-(2,3-dimethyl-phenyl)-methanol (HCl) by removal of the benzyl group and reduction of the hydroxyl group with hydrogen (H₂/Pd-C in HCl), extraction with ethyl acetate in the presence of ammonium hydroxide, addition of hydrogen chloride (HCl) followed by cooling to induce precipitation and washing and drying of the resulting raw product;
b) dissolving said raw product in water under heating until a clear solution is obtained;
c) cooling said clear solution, which results in the formation of a crystalline precipitate;
d) separating said crystals from the solution and washing said crystals with water;
e) drying said precipitate under vacuum to obtain detomidine hydrochloride monohydrate.

Another object of the present invention is detomidine hydrochloride monohydrate for use as a medicament.

Another object of the present invention is detomidine hydrochloride monohydrate for use as anesthetic, analgesic, muscle relaxant, sedative and any other application susceptible to treatment with an alpha₂-adrenoreceptor agonist in a mammal. According to the invention detomidine hydrochloride monohydrate can be used for the treatment of animals like horses or cattle or other mammal animals and for the treatment of humans.

A further object of the present invention is a pharmaceutical composition for use as anesthetic, analgesic, muscle relaxant, sedative and any other application susceptible to treatment with an alpha₂-adrenoreceptor agonist in a mammal, comprising detomidine hydrochloride monohydrate, in combination with pharmaceutically acceptable excipients.

Especially preferred according to the present invention is a pharmaceutical composition, wherein the employed detomidine hydrochloride monohydrate has a water content in the range of 7.2 % to 7.8 % by weight, preferred in the range of 7.4 % to 7.6 % by weight.

Further preferred is a pharmaceutical composition, wherein the pharmaceutical composition is in form of a parenterally applicable formulation.
Further preferred is also a pharmaceutical composition, wherein the pharmaceutical composition is in form of an orally applicable formulation.
Further preferred is finally a pharmaceutical composition, wherein the pharmaceutical composition is in form of a topically applicable formulation.

This means that detomidine hydrochloride monohydrate is suitable for any kind of application to a mammal, like a human.

Another object of the present invention is a pharmaceutical composition comprising detomidine hydrochloride monohydrate as active ingredient, obtainable by adding detomidine hydrochloride monohydrate to pharmaceutically acceptable excipients and mixing the same, producing a mixture of detomidine hydrochloride monohydrate and said pharmaceutically acceptable excipients and converting said mixture into a pharmaceutical composition in a form that can be applied to a mammal parenterally, orally or topically.

Just another object of the present invention is a method for the preparation of detomidine hydrochloride monohydrate comprising the following steps:
a) providing detomidine hydrochloride raw product, wherein the raw product is synthesized from (RS)-(3-Benzyl-3*H*-imidazol-4-yl)-(2,3-dimethyl-phenyl)-methanol (HCl) by removal of the benzyl group and reduction of the hydroxyl group with hydrogen (H₂/Pd-C in HCl), extraction with ethyl acetate in the presence of ammonium hydroxide, addition of hydrogen chloride followed by cooling to induce precipitation and washing and drying of the resulting raw product;
b) dissolving said raw product in water under heating until a clear solution is obtained;
c) cooling said clear solution, which results in the formation of a crystalline precipitate;
d) separating said crystals from the solution and washing said crystals with water;
e) drying said precipitate under vacuum to obtain detomidine hydrochloride monohydrate.

A further object of the present invention is the use of detomidine hydrochloride monohydrate or a pharmaceutical composition comprising detomidine hydrochloride monohydrate for anesthesia, analgesia, muscle relaxation or sedation in a mammal.

### Brief Description of Drawings

The present invention is described in detail also with the present drawings and figures.
Figure 1 shows the DSC curve (50 - 180 °C, 10 K/min) of the detomidine hydrochloride monohydrate (DHM) batch RSc 101101 K1a after storage over 36 + 64 months (see Example 6);
Figure 2 shows the DSC curve of DHM batch MBa 120112 K1 after storage over 48 + 26 months (see Example 7);
Figure 3 shows the DSC curve measurement of a detomidine hydrochloride batch with a water content (KF) of 1.71 % (see Example 8);
Figure 4 shows a typical PXRD spectrum of a detomidine hydrochloride monohydrate batch;
Figure 5 shows the microscopic appearance of DHM batch NK E-190709-I A K1 (see Example 2) before (Fig. 5a) and after (Fig. 5b) grinding; and
Figure 6 shows the HPLC chromatogram of this DHM.

### Description of Embodiments

In contrast to anhydrous detomidine hydrochloride known in the prior art (i.e. as defined in the EUROPEAN PHARMACOPOEIA) which was shown to represent a hydrate mixture which is susceptible to unpredictable changes in water content during storage, detomidine hydrochloride monohydrate according to the present invention represents the pure monohydrate which in contrast to the prejudice in the prior art does not show any significant conversion to the anhydrate or changes in other relevant specification parameters during storage.

Detomidine hydrochloride monohydrate can reproducibly be produced by a novel method by using water for the final recrystallization process. This new and simple production method leads to a stable and very pure product that can advantageously be used for the preparation of pharmaceutical compositions for the treatment of different pathological conditions susceptible to treatment with an alpha₂-adrenoreceptor agonist.

Surprisingly it was found that detomidine hydrochloride monohydrate according to the invention does not only show a HPLC purity that is much higher than that required for anhydrous detomidine hydrochloride by the EUROPEAN PHARMACOPOEIA (Ph. Eur. 9.0) but also shows an extremely low content of residual solvents (i.e. ≤100 ppm or even ≤50 ppm for any of the detected single solvents).

The pure hydrate form and high purity of detomidine hydrochloride monohydrate according to the invention also leads to a surprisingly high storage stability of at least 48 months.

The named advantages of the present invention are explained in detail below and in the following examples.

Due to the hygroscopicity of anhydrous detomidine hydrochloride (Ph. Eur. 9.0) the substance avidly takes up water from the atmosphere or process solutions containing water so that it is difficult to obtain absolutely water-free (i.e. anhydrous) detomidine hydrochloride during synthesis/production in a normal production environment (i.e. without humidity controls). Typically, the obtained detomidine hydrochloride raw products and corresponding purified crystallizates contain significant amounts of water which often even result in yields above 100 % of the theory (i.e. for the anhydrous material). This water has to be removed during final drying using harsh conditions (i.e. elevated temperatures and vacuum (as needed)). Furthermore, the dried product has to be rapidly filled into airtight containers to avoid uptake of atmospheric water. Under these conditions it proved difficult to meet the former "water limit" of ≤ 0.5 % (Ph. Eur. 8.0) even immediately after production of the drug substance (i.e. during release testing). These problems are most likely the reason why the potential water content of "anhydrous" detomidine hydrochloride allowed by the respective monograph was increased from ≤ 0.5 % (Ph. Eur. 8.0) to ≤ 2.0 % (first introduced in Ph. Eur. 8.4).
In contrast to this, detomidine hydrochloride monohydrate (DHM) according to the present invention can reproducibly be synthesized in a standard production environment under ambient conditions without the need for harsh drying conditions, elaborate handling or humidity controls. Furthermore, use of airtight containers for storage is not required.

Detomidine hydrochloride with a water content (KF) of up to 2.0 % (Ph. Eur. 9.0) does not represent pure anhydrous detomidine hydrochloride but rather a mixture of the monohydrate and anhydrate. Thus, detomidine hydrochloride with a water content of exactly 2.0 % theoretically contains 26.7 % of the monohydrate form. Such potentially high content of the "wrong" hydrate form is typically undesirable for a well-defined drug substance in the pharmaceutical setting. Thus, it is known that hydrates typically display different physical properties (e. g. solubility and dissolution rate) compared to the anhydrous form which may have a marked impact on the bioavailability of the respective drug substance and corresponding drug product. Based on the current Ph. Eur. specification which potentially allows a water content of up to 2.0 % one may initially use anhydrous detomidine hydrochloride which may partly convert to the monohydrate form during storage of the drug substance or storage of the corresponding drug product which may have a negative effect on product quality.

DHM according to the present invention does not represent a mixture of different hydrate forms but rather the pure monohydrate. Additionally, it does not show any significant conversion to the anhydrate during storage.

The increase in the allowed water content for detomidine hydrochloride to ≤ 2.0 % (Ph. Eur. 8.4) is apparently based on the assumption that the water content of anhydrous detomidine can be adequately and consistently controlled when taking corresponding measures during production and storage (e.g. use of airtight containers) so that the corresponding specification limit of ≤ 2.0 % (Ph. Eur. 9.0) can consistently be met over the intended duration of use. However, it was surprisingly found that detomidine hydrochloride stored in airtight containers under the long-term conditions as required by ICH Q1A (i.e. storage at 25 °C and 60 % relative humidity (RH)) may unpredictably exceed the water limit at later time points. As can be seen in Table 1, one batch produced at a time when Ph. Eur. 8.0 was in force which initially met all requirements of the corresponding monograph fell dramatically outside of the specification regarding loss on drying after 36 months of storage. At that point in time the loss on drying amounted to 7.51 % which is in the range of the theoretical water content of the monohydrate. Already at earlier time points a marked increase in loss on drying (i.e. water content) was observed and the loss on drying after 24 months which amounted to 0.40 % was already only slightly below the corresponding specification limit of ≤ 0.5 % valid at that time.
Similar results were obtained for another batch produced at a time when Ph. Eur. 8.0 was in force and where a change in the monograph (i.e. Ph. Eur. 8.4) occurred during long-term storage. After 36 months of storage (see Table 2) the water content of the tested stability sample amounted to 1.32 % which corresponded quite well with the loss on drying of 1.37 % determined at the same point in time (to allow comparison to the former values obtained in line with the requirement of Ph. Eur. 8.0). However, after 48 months the tested stability sample unpredictably showed a water content of 7.54 % well above the corresponding Ph. Eur. specification limit of ≤ 2.0 %.

Consequently, it cannot be excluded that detomidine hydrochloride as specified by the current Ph. Eur. will transform partly or completely to the monohydrate during storage of the drug substance so that one has to repeat determination of the water content immediately prior to the planned use for drug product production to make sure the correct amount of drug substance is weighed-in to avoid a shortfall in the assay (i.e. content of drug substance) in the final pharmaceutical preparation. More importantly, however, this change in water content which may also occur during production of the drug substance and/or storage of the corresponding drug products may have a significant effect on their physico-chemical properties (e.g. solubility or dissolution behavior) and resulting bioavailability.
DHM according to the present invention does not show any significant changes in water content or other relevant specification parameters during storage. Consequently, no changes in physico-chemical properties of the corresponding drug products (i.e. pharmaceutical preparations) which could be caused by the employed drug substance are to be expected.

In summary, there is a prejudice in the prior art that the anhydrous form of detomidine hydrochloride as specified by the Ph. Eur. and currently used in all commercially available drug products is particularly suitable for pharmaceutical use while in fact it is less suitable than the monohydrate form due for example to the challenges involved in its reproducible production and its susceptibility to water uptake during storage.
Additionally, there is a prejudice that the anhydrous form and the monohydrate form can easily interconvert, depending on ambient temperature and air humidity, while in fact the monohydrate form is stable under ambient as well as ICH storage conditions and does not show any significant interconversion during processing and storage.

Detomidine hydrochloride monohydrate (DHM) according to the present invention can be produced by the various methods for preparation of hydrates known to those skilled in the art (i.e. from the synthesis other monohydrate compounds) may be used.
More specifically, the monohydrate may be obtained by treatment of anhydrous detomidine hydrochloride (i.e. water content ≤ 2.0 %) with water or humid air as described earlier.
Alternatively, the monohydrate may be formed during the last step of the detomidine synthesis by addition of the required amount of water during a suitable stage of the overall synthetic procedure (e.g. during crystallization or purification).

In a preferred embodiment of the present invention DHM is reproducibly obtained in high yields (i.e. ≥ 75 % or most preferred ≥ 85 %) by recrystallization of the initial detomidine hydrochloride raw product from the last step of the overall synthesis from water (e.g. potable water, demineralized water or water for injection). Most preferred is the use of water for injection for this recrystallization step.
Surprisingly, it was found that such recrystallization from water (instead of recrystallization from organic solvents as typically performed) and subsequent washing with water or low-chain alcohols (i.e. methanol, ethanol, n-propanol or 2-propanol) alone or in mixtures followed by drying under mild conditions will not only result in reproducible formation of the monohydrate but will also result in a very high HPLC purity as well as an extremely low residual solvent concentration in the corresponding final drug substance.

The final drug substance can be dried to remove residual solvents (incl. excess water) at room temperature or at elevated temperatures (≤ 45 °C) and/or under vacuum (≤ 100 mbar). Most preferred is drying at 20 ± 5 °C and a vacuum of ≤ 20 mbar.
The resulting detomidine hydrochloride monohydrate shows a water content between 6.8 % and 8.2 %, more preferred between 7.2 and 7.8 % or most preferred between 7.4 and 7.6 %.

When studied by PXRD detomidine hydrochloride monohydrate according to the present invention shows the typical PXRD patterns already known from the art.
If measured by differential scanning calorimetry (DSC, e.g. at 10 K/min in a temperature range between 50 - 180 °C) detomidine hydrochloride monohydrate according to the present invention will show a first peak with an onset around 96 °C which represents loss of water and a second main peak with an onset around 160 °C which represents melting of detomidine hydrochloride. The first peak is completely absent in anhydrous detomidine hydrochloride while it will be clearly visible in drug substance containing water in the range as allowed by the current Ph. Eur. (i.e. ≤ 2.0 %).
In preferred cases no new/additional impurities are formed during preparation of the monohydrate and the first crystallizate (K1) already shows an acceptable purity. Alternatively, recrystallization can be repeated until the desired purity is obtained.
DHM according to the present invention shows a HPLC purity of ≥ 99.5 % with a content of ≤ 0.20 % for any (unspecified) impurities and ≤ 0.5 % for total impurities (during release and storage). In preferred cases, the resulting DHM shows a HPLC purity ≥ 99.8 % with a content of ≤ 0.10 % for any impurity (i.e. specified and unspecified) and ≤ 0.25 % for total impurities. In most preferred cases, there are no unknown impurities above 0.05 % and the total content of impurities is ≤ 0.20 or even ≤ 0.10 %. The respective limits are met during initial release testing as well as during (ongoing) stability testing of the drug substance over the desired period of use.

DHM according to the present invention does not contain any of the residual solvents listed in ICH Q3C at levels above 30 % or more preferred 10 % of the respective concentration limit from this ICH guideline (i.e. in its most recent version). In preferred cases the resulting DHM does not contain any organic solvent other than the one used during the last purification step (-if any) at a concentration ≥ 100 ppm or even more preferred ≥ 50 ppm. Most preferred is the absence of any residual solvents in the final DHM (i. e. no solvent found above the LOQ or even LOD of the employed GC method (GC-FID or -MS)).

The last step of the synthesis of detomidine hydrochloride monohydrate according to the present invention can be performed under ambient conditions (i.e. under normal atmospheric conditions) in a standard production environment typically used for drug substances without requiring specific handling (e.g. limitation of the duration of working steps with exposure to ambient humidity) as necessary for the hygroscopic anhydrous form. Thus, no elaborate controls or adjustment of the relative humidity are required during manufacture of either the drug substance itself or the corresponding drug products (incl. synthesis/formulation, drying, testing, packaging and storage (e.g. warehouse)).

Detomidine hydrochloride monohydrate (DHM) according to the present invention may be stored in standard containers under ambient conditions or under the conditions as prescribed by ICH Q1A over prolonged time periods without any significant changes. Thus, the substance is stable (i.e. meets the pertinent specification requirements) at 25 °C and 60 % relative humidity over at least 36 months, more preferred over at least 48 months or most preferred over at least 60 months. The substance is also stable during accelerated stability testing at 40 °C and 75 % relative humidity over at least 6 months more preferably over at least 12 months.

Detomidine hydrochloride monohydrate (DHM) according to the present invention may advantageously be used in pharmaceutical preparations (i.e. drug products/formulations) for veterinary or human use. In preferred cases DHM is used in drug products for veterinary use. Most preferred is the use in drug products for human use.

DHM according to the present invention may be used in pharmaceutical preparations for e.g. parenteral (e.g. intravenous, subcutaneous, intramuscular), oral (incl. sublingual), topical (e.g. dermal, ocular) or other (e.g. intraocular, intravaginal) routes of application. In preferred cases DHM is used in pharmaceutical preparations for parenteral or oral use.

DHM can be formulated e.g. into tablets, pellets, capsules, solutions, creams, gels, nano- or microcapsules, liposomes, micelles, implants or any other formulation type (i.e. pharmaceutical preparation or drug product) known to those skilled in the art and described for example in Hager's Handbuch [Handbook] (5th ed.) 2, 622-1045; List et al., Arzneiformenlehre [Instructions for Drug Forms], Stuttgart: Wiss. Verlagsges. 1985; Sucker et al., Pharmazeutische Technologie [Pharmaceutical Technology], Stuttgart: Thieme 1991; Ullmann's Enzyklopädie [Encyclopedia] (5th ed.) A 19, 241-271; Voigt, Pharmazeutische Technologie [Pharmaceutical Technology], Berlin: Ullstein Mosby 1995.
The pharmaceutical DHM preparations according to the present invention may contain the drug substance in partly or completely dissolved form (i.e. use of a solution). In more preferred cases DHM is partly or completely present in suspended form (i.e. use of a suspension).

DHM containing pharmaceutical compositions/preparations (drug products) according to the present invention may be used for anesthesia, analgesia, muscle relaxation, sedation and any other indication/condition susceptible to treatment with an alpha₂-adrenoreceptor agonist.
In summary, use of detomidine hydrochloride monohydrate according to the present invention offers the following advantages:
- Can reproducibly be synthesized with high yields
- Can be produced under ambient conditions in a standard production environment as typically used for drug substances so that no elaborate handling or controls (i.e. humidity) are required
- Does not require use of organic solvents during the last purification (i.e. recrystallization step)
- Displays a very high purity (i.e. low content of organic impurities as determined by HPLC and residual solvents as determined by GC)
- Does not represent a mixture of the anhydrous and monohydrate form
- Does not require any sophisticated container or other aids to assure storage stability of the drug substance
- Does not show any (significant) interconversion to the anhydrous form during processing and storage
- Does not show any significant changes in water content during normal storage conditions (as defined by the pertinent pharmacopoeias or other regulations) so that no retests prior to use (i.e. manufacture of the corresponding drug product) are needed to determine the content as is
- Is stable over prolonged time periods consistently meeting all specification requirements (incl. purity, loss on drying and/or water content as applicable)
- Can advantageously be used in pharmaceutical preparations (i.e. drug products) for veterinary or human use.

The present invention is explained in detail referring to the enclosed figures and tables. The present invention is also described in detail with the following examples, which are not intended to limit the scope of the invention.

### Example 1

### Preparation of detomidine hydrochloride monohydrate (DHM)

Detomidine hydrochloride was synthesized starting from 1-benzyl-imidazole-4-carboxyaldehyde and 2,3-dimethylphenlymagnesiumbromide according to the two-step synthesis described in Drugs Future 10, 17 (1985).

For the second step of this synthesis (RS)-(3-Benzyl-3*H*-imidazol-4-yl)-(2,3-dimethyl-phenyl)-methanol (HCl) was suspended in a mixture of water and hydrochloric acid. The catalyst (i. e. palladium on activated carbon) suspended in demineralized water was added. Hydrogenation (i.e. removal of the benzyl group and reduction of the hydroxyl group with hydrogen (H₂/Pd-C in HCl)) was performed at elevated temperature (50 - 80 °C) and the obtained suspension was filtered after the hydrogenation was finished. Subsequently ethyl acetate and a solution of ammonium hydroxide were added under continuous stirring. After discontinuation of stirring, phase separation occured after which the aqueous phase was repeatedly extracted with ethyl acetate. The combined organic phases were washed with demineralized water and filtered.

After addition of 5 - 6 N hydrogen chloride in 2-propanol and cooling precipitation of detomidine hydrochloride occured. After filtration the filtercake (i.e. raw product) was washed with ethyl acetate and dried.

A fraction of the resulting raw product (i.e. 5 g batch RSO E-190604 RP) was recrystallized from 5 g demineralized water by heating (until complete dissolution was obtained) and subsequent cooling on an ice bath. The resulting crystals were separated by filtration and the resulting filter cake washed with 2-propanol. Subsequently, the washed product was dried under vacuum (10 mbar) at 23 °C. The obtained yield for the white crystalline substance amounted to 66.0 % of the theory.

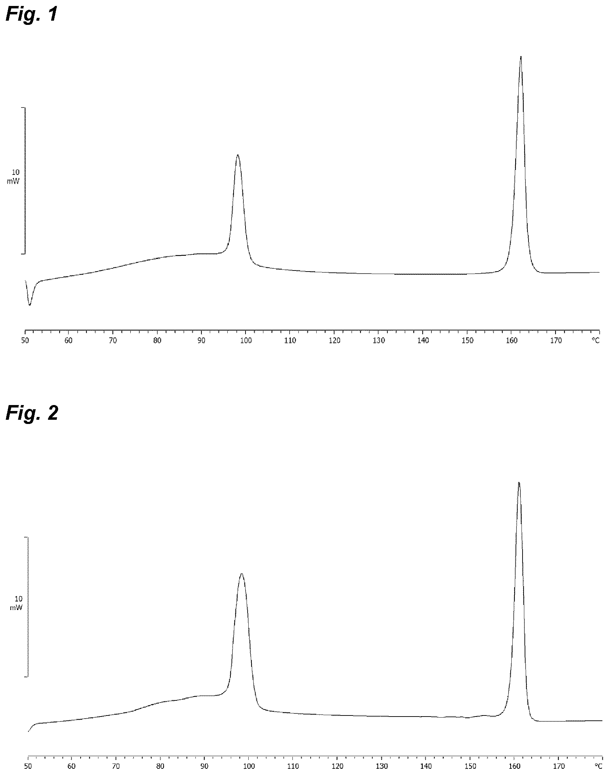

The resulting drug substance showed a water content (KF) of 7.49 %. The corresponding DSC curve was in line with the expectation (see for example Figure 1) and showed the two typical peaks routinely observed for DHM. Other than 2-propanol used for final washing none of the other solvents employed during the overall synthesis of this compound were found above the respective LOQ by GC-FID.

### Example 2

### Impurities after preparation of detomidine hydrochloride monohydrate (DHM)

A larger batch of detomidine hydrochloride (i.e. 50 g NK E-190709-I A K1) was synthesized in line with Example 1. However, the final crystals obtained after recrystallization from 50 ml demineralized water were washed with 25 ml demineralized water instead of 2-propanol. Drying was performed at 21 °C and 10 mbar until constant weight. The obtained yield for the white crystalline substance amounted to 87.2 % of the theory which was markedly higher than the yield obtained in Example 1. The water content of this substance was determined at 7.54 % (KF) and the corresponding DSC curve showed two peaks with an onset at 95.7 °C and 159.3 °C.

As shown below, recrystallization of the initial raw product from water (incl. washing) resulted in significant removal/reduction of impurities eluting before the detomidine peak (i.e. more polar compounds, e.g. Impurity A) as well as impurities eluting behind the detomidine peak (i.e. less polar compounds, e.g. Impurity C).

| **Sample** | **Relevant compounds as detected by HPLC [area%]*** | | | | |
|---|---|---|---|---|---|
| | **Impurity A** | **Impurity RRT 0.84** | **Detomidine** | **Impurity RRT 1.75** | **Impurity C** |
| Raw product | 0.11 | 0.33 | 99.40 | 0.04 | 0.04 |
| Final crystallizate (K1) | 0.06 | 0.06 | 99.80 | 0.01 | 0.02 |

| | | | | | |
|---|---|---|---|---|---|
| *Table includes all compounds found at or above 0.04 area% in the initial raw product in the order in which they eluted from the HPLC column | | | | | |

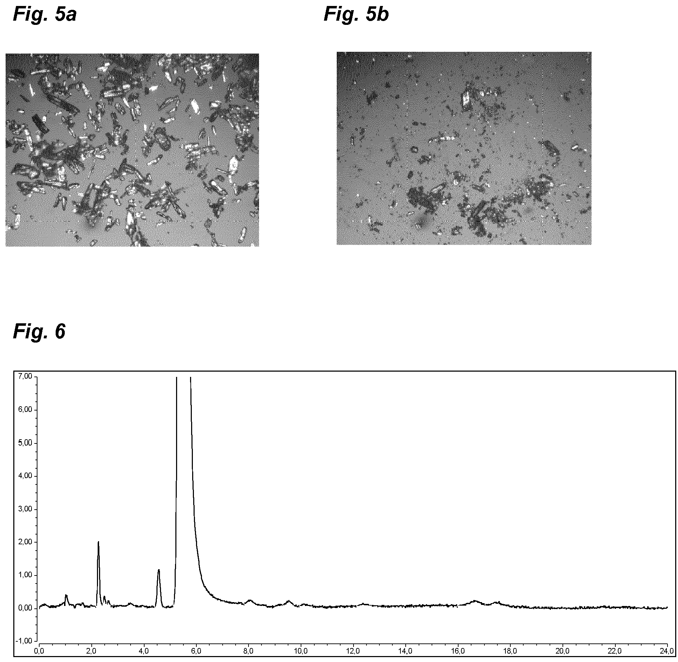

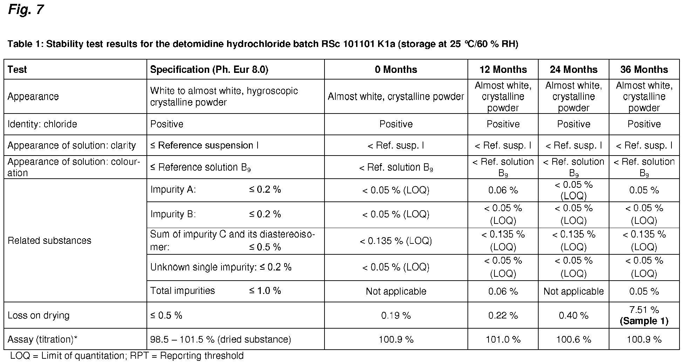

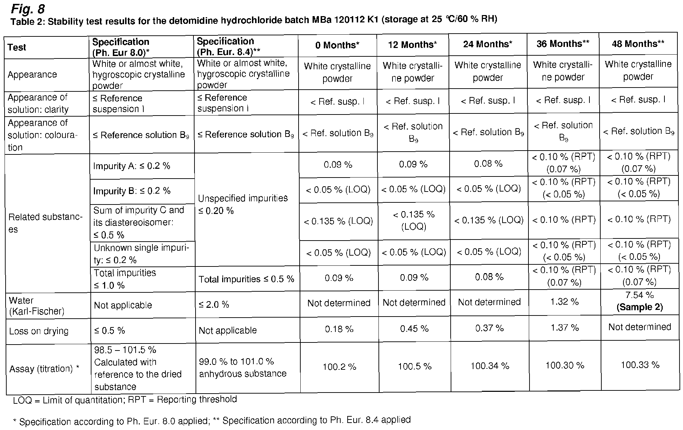

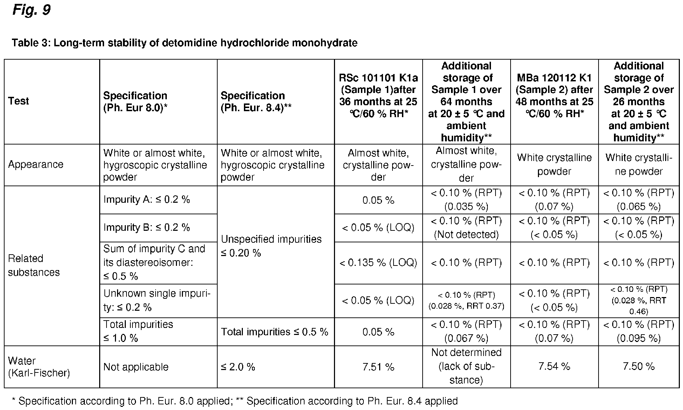

The final substance showed a very high HPLC purity of 99.80 area% (Ph. Eur. test method) and only a limited number of unknown impurities in addition to those listed in the table above at concentrations « 0.02 area%. The corresponding HPLC chromatogram obtained after manual integration of all peaks is presented in Figure 6.

### Example 3

### Hygroscopicity of anhydrous detomidine hydrochloride

The hygroscopicity of anhydrous detomidine hydrochloride was studied based on the corresponding method provided in the Ph. Eur. (section 5.11.). To this end approximately 1 g of batch JL 131211 K1 which had been dried to constant weight in a glass weighing vessel were placed in a desiccator at 25 °C containing a saturated solution of ammonium chloride and allowed to stand for 6 days. The weight increase was monitored as described by the Ph. Eur. After 24 hours the increase amounted to 2.8 % so that this substance falls into the category of hygroscopic substances according to the Ph. Eur. After 4 days the weight increase amounted to 7.3 % and after 6 days to 7.4 % the latter of which is close to the theoretical water content of the monohydrate of 7.5 %.

### Example 4

### Long-term stability of anhydrous detomidine hydrochloride

The long-term stability of anhydrous detomidine hydrochloride was studied at 25 °C and 60 % relative humidity. The stability samples were stored in tightly closed 30 ml brown glass (type III, Ph. Eur.) containers with screw thread. These containers were closed with black polypropylene screw caps with an inner LDPE sealing disk and additionally sealed with PARAFILM® to assure airtightness.

Stability was tested in the prescribed interval and the corresponding results are shown in Table 1.

### Example 5

### Long-term stability of anhydrous detomidine hydrochloride

Another long-term stability study was performed in line with Example 4. The corresponding results are shown in Table 2.

### Example 6

### Stability sample 1 (batch RSc 101101 K1a)

The stability sample from the detomidine hydrochloride batch RSc 101101 K1a which showed a water content of 7.51 % **(Sample 1)** after 36 months of storage at 25 °C and 60 % relative humidity (see Table 1) was subsequently stored in the same container (additionally sealed with PARAFILM®) at 20 ± 5 °C and ambient humidity for another 64 months and tested regarding its polymorphic form (DSC) and HPLC purity (as per Ph. Eur. 8.4).

As shown Figure 1 the substance still exists in the monohydrate form. The first endothermic peak with an onset around 96 °C represents loss of water and the second peak with an onset around 160 °C represents melting of detomidine hydrochloride.

As shown in Table 3 the HPLC purity **(Sample 1)** remained unchanged over 64 months demonstrating the excellent storage stability of detomidine hydrochloride monohydrate.

### Example 7

### Stability sample 2 (batch MBa 120112 K1)

The stability sample from the detomidine hydrochloride batch MBa 120112 K1 which showed a water content of 7.54 % **(Sample 2)** after 48 months of storage at 25 °C and 60 % relative humidity (see Table 2) was subsequently stored for another 26 months as described above (see Example 6).

In addition to DSC and HPLC purity testing the water content of the sample was determined by Karl Fischer (KF) titration. The corresponding DSC curve is shown in Figure 2 and the other analytical results in Table 3.

It has to be noted that the determined water content of 7.5 % (KF) represents the theoretical water content of the monohydrate.

### Example 8

### DSC measurement of anhydrous detomidine hydrochloride

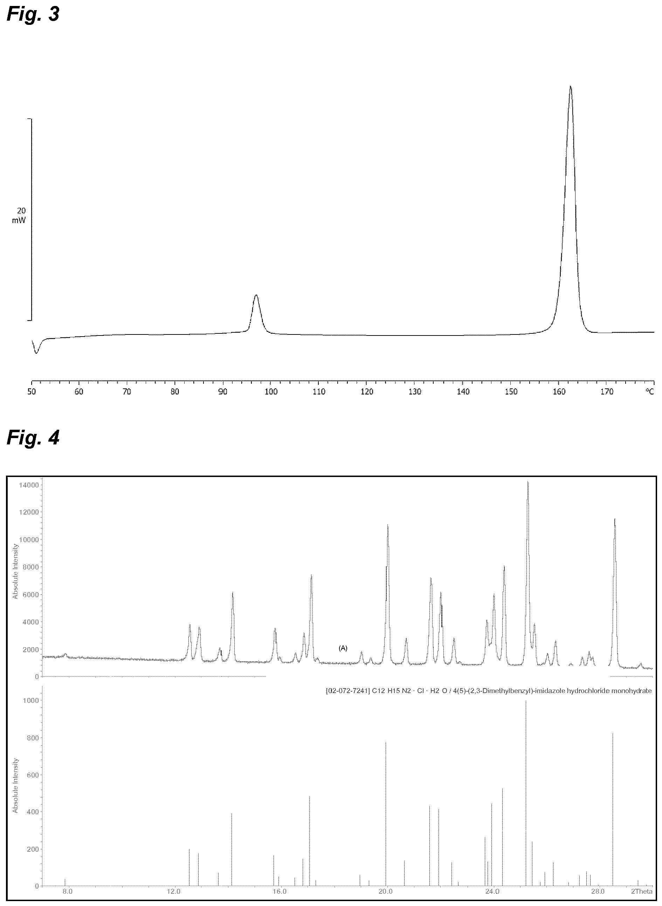

The DSC curve of an "anhydrous" detomidine hydrochloride sample which meets the specification requirements of the Ph. Eur. 8.4 is shown in Figure 3. The water content of this sample as determined by Karl Fischer titration amounted to 1.71 %. As can be seen in the DSC curve the substance represents a mixture of anhydrous detomidine hydrochloride and the corresponding monohydrate (DHM content approximately 22.8 % based on the determined water content).

### Example 9

### Powder X-ray diffraction spectrum

A typical PXRD spectrum of detomidine hydrochloride monohydrate obtained by treatment of anhydrous detomidine hydrochloride with humid air over night (see Example 4) is presented in Figure 4. The spectrum corresponds with the published PXRD-data for DHM from the state of the art.

### Example 10

Residual solvent content after preparation of detomidine hydrochloride

### monohydrate (DHM)

The DHM batch obtained from Example 2 was analyzed regarding those solvents used during its overall synthesis by GC-FID. The following results were obtained:

| **Employed solvent** | **Residual solvent concentration of the final drug substance [ppm]** | **ICH Q3C concentration limit [ppm]** |
|---|---|---|
| Acetone | < 12 (LOQ) | 5000 |
| Dichloromethane | < 28 (LOD) | 600 |
| | (86 = LOQ) | |
| Ethyl acetate | < 22 (LOQ) | 5000 |
| 2-Propanol | < 42 (LOQ) | 5000 |
| Tetrahydrofuran | < 16 (LOQ) | 720 |
| Toluene | < 18 (LOQ) | 890 |

### Example 11

### Particle properties of detomidine hydrochloride monohydrate (DHM)

The particle appearance and size of the DHM batch obtained from Example 2 was determined by polarized light microscopy and laser diffraction (Malvern MASTERSIZER 2000) before and after manual grinding of approximately 12 g over 2 min in a porcelain mortar with a porcelain pistil.

The microscopic results are presented in Figure 5.

The laser light diffraction measurements gave the following results for the original substance (d (v/0.1) = 54 µm, d (v/0.5) = 162 µm and d (v/0.9) = 532 µm) and the corresponding substance after grinding (d (v/0.1) = 25 µm, d (v/0.5) = 97 µm and d (v/0.9) = 308 µm).

### Example 12

### Stress stability of detomidine hydrochloride monohydrate (DHM)

In order to simulate accelerated stability testing according to ICH Q1A over 6 months at 40 °C and 75 % relative humidity the DHM batch obtained from Example 2 was subjected to stress stability testing at 80 °C and 75 % RH over 12 days (i.e. approximate kinetically equivalent conditions).

As shown below no changes in HPLC purity (Ph. Eur. test method) could be observed under these stress conditions.

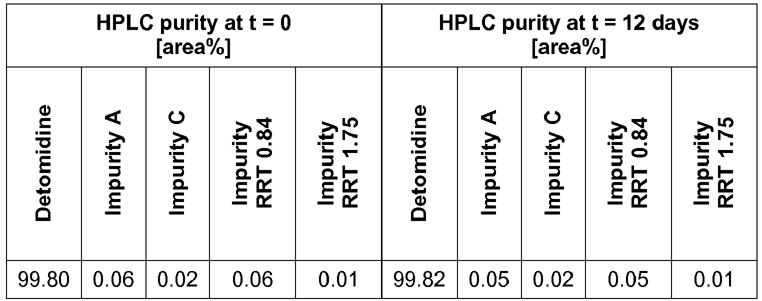

## Claims

1. Detomidine hydrochloride monohydrate, wherein the water content is in the range of 7.2 to 7.8 % (w/w).

2. Detomidine hydrochloride monohydrate, according to claim 1, wherein the water content is in the range of 7.4 to 7.6 % (w/w).

3. Detomidine hydrochloride monohydrate, according to claim 1 or 2,
wherein the residual solvent content is less than 100 ppm for any of the detected single solvents.

4. Detomidine hydrochloride monohydrate, according to claim 3, wherein the residual solvent content is equal to or less than 50 ppm for any of the detected single solvents.

5. Detomidine hydrochloride monohydrate, according to at least one of the preceding claims, wherein the HPLC purity is equal to or higher than 99.8 %.

6. Detomidine hydrochloride monohydrate, according to at least one of the preceding claims, wherein the content of (RS)-(2,3-dimethylphenyl) (1H-imidazol-4-yl)-methanol, (RS)-(1-benzyl-1H-imidazol-5-yl) (2,3-dimethylphenyl)-methanol and 4-[(2,3-dimethylcylohexyl) methyl]-1H-imidazole as impurities is less than 0.1 % each.

7. Detomidine hydrochloride monohydrate, according to at least one of the preceding claims, wherein the storage stability at 25 °C and 60 % relative humidity is equal to or higher than 36 months.

8. Detomidine hydrochloride monohydrate, according to claim 7, wherein the storage stability at 25 °C and 60 % relative humidity is equal to or higher than 48 months.

9. Detomidine hydrochloride monohydrate, obtainable by
a) providing detomidine hydrochloride raw product, wherein the raw product is synthesized from (RS)-(3-Benzyl-3*H*-imidazol-4-yl)-(2,3-dimethyl-phenyl)-methanol (HCl) by removal of the benzyl group and reduction of the hydroxyl group with hydrogen (H₂/Pd-C in HCl), extraction with ethyl acetate in the presence of ammonium hydroxide, addition of hydrogen chloride (HCl) followed by cooling to induce precipitation and washing and drying of the resulting raw product;
b) dissolving said raw product in water under heating until a clear solution is obtained;
c) cooling said clear solution, which results in the formation of a crystalline precipitate;
d) separating said crystals from the solution and washing said crystals with water;
e) drying said precipitate under vacuum to obtain detomidine hydrochloride monohydrate.

10. Detomidine hydrochloride monohydrate for use as a medicament.

11. Detomidine hydrochloride monohydrate for use as anesthetic, analgesic, muscle relaxant, sedative and any other application susceptible to treatment with an alpha₂-adrenoreceptor agonist in a mammal.

12. A pharmaceutical composition for use as anesthetic, analgesic, muscle relaxant, sedative and any other application susceptible to treatment with an alpha₂-adrenoreceptor agonist in a mammal, comprising detomidine hydrochloride monohydrate, in combination with pharmaceutically acceptable excipients.

13. The pharmaceutical composition according to claim 12, wherein the employed detomidine hydrochloride monohydrate has a water content in the range of 7.2 % to 7.8 % by weight, preferred in the range of 7.4 % to 7.6 % by weight.

14. The pharmaceutical composition, according to at least one of the claims 12 and 13, **characterized in that** the pharmaceutical composition is in form of a parenterally applicable formulation.

15. The pharmaceutical composition, according to at least one of the claims 12 and 13, **characterized in that** the pharmaceutical composition is in form of an orally applicable formulation.

16. The pharmaceutical composition, according to at least one of the claims 12 and 13, **characterized in that** the pharmaceutical composition is in form of a topically applicable formulation.

17. A pharmaceutical composition comprising detomidine hydrochloride monohydrate as active ingredient, obtainable by adding detomidine hydrochloride monohydrate to pharmaceutically acceptable excipients and mixing the same, producing a mixture of detomidine hydrochloride monohydrate and said pharmaceutically acceptable excipients and converting said mixture into a pharmaceutical composition in a form that can be applied to a mammal parenterally, orally, or topically.

18. A method for the preparation of detomidine hydrochloride monohydrate comprising the following steps:
a) providing detomidine hydrochloride raw product, wherein the raw product is synthesized from (RS)-(3-Benzyl-3*H*-imidazol-4-yl)-(2,3-dimethyl-phenyl)-methanol (HCl) by removal of the benzyl group and reduction of the hydroxyl group with hydrogen (H₂/Pd-C in HCl), extraction with ethyl acetate in the presence of ammonium hydroxide, addition of hydrogen chloride followed by cooling to induce precipitation and washing and drying of the resulting raw product;
b) dissolving said raw product in water under heating until a clear solution is obtained;
c) cooling said clear solution, which results in the formation of a crystalline precipitate;
d) separating said crystals from the solution and washing said crystals with water;
e) drying said precipitate under vacuum to obtain detomidine hydrochloride monohydrate.
